Europäisches Patentamt

European Patent Office

Office européen des brevets

(19)

(11) Veröffentlichungsnummer : **0 127 073**
**B1**

(12) # EUROPÄISCHE PATENTSCHRIFT

(45) Veröffentlichungstag der Patentschrift :
**19.08.87**

(21) Anmeldenummer : **84105593.2**

(22) Anmeldetag : **16.05.84**

(51) Int. Cl.⁴ : **A 61 B   6/00**, A 61 B   6/04,
H 05 G   1/02

(54) **Röntgendiagnostikgerät für Mammographieaufnahmen.**

(30) Priorität : **30.05.83 DE 3319622**

(43) Veröffentlichungstag der Anmeldung :
**05.12.84 Patentblatt 84/49**

(45) Bekanntmachung des Hinweises auf die Patenterteilung : **19.08.87 Patentblatt 87/34**

(84) Benannte Vertragsstaaten :
**BE DE FR**

(56) Entgegenhaltungen :
**EP-A- 0 075 831**
**WO-A-80 /011 11**
**BE-A-   811 497**
**FR-A- 1 513 676**
**FR-A- 2 352 531**
**US-A- 1 698 895**
**US-A- 2 545 899**
**US-A- 3 549 885**

(73) Patentinhaber : **Siemens Aktiengesellschaft Berlin**
**und München**
**Wittelsbacherplatz 2**
**D-8000 München 2 (DE)**

(72) Erfinder : **Dornheim, Hans-Peter**
**Meisenweg 15**
**D-8521 Bubenreuth (DE)**
Erfinder : **Saffer, Edmund**
**Pestalozzistrasse 4**
**D-8551 Eggolsheim (DE)**

## Beschreibung

Die Erfindung betrifft ein Röntgendiagnostikgerät für Mammographieaufnahmen gemäß dem Oberbegriff des Patentanspruches 1.

Es ist ein Röntgendiagnostikgerät dieser Art bekannt (BE-A-811 497), bei dem mehrere unterschiedliche Aufnahmetische, insbesondere mit unterschiedlichen Formaten, vorgesehen sind, die wahlweise an dem Röntgenstrahler befestigbar sind. Zur Anfertigung einer Mammographieaufnahme wählt demgemäß der Arzt den gewünschten Aufnahmetisch aus, befestigt diesen am Röntgenstrahler und löst dann die Aufnahme aus. Die Verbindung des jeweils gewünschten Aufnahmetisches mit dem Röntgenstrahler ist dabei relativ zeitaufwendig. Ferner ist es erforderlich, die nicht benutzten Aufnahmetische gesondert vom Röntgendiagnostikgerät aufzubewahren.

Der Erfindung liegt die Aufgabe zugrunde, ein Röntgendiagnostikgerät der eingangs genannten Art zu schaffen, bei dem eine gesonderte Aufbewahrung der unterschiedlichen Aufnahmetische nicht erforderlich und eine einfache Einbringung des jeweils gewünschten Aufnahmetisches in die Aufnahmeposition möglich ist.

Diese Aufgabe ist erfindungsgemäß gelöst durch die Merkmale des kennzeichnenden Teiles des Anspruches 1. Das erfindungsgemäße Röntgendiagnostikgerät trägt die beiden für Röntgenaufnahmen vorgesehenen Aufnahmetische, die durch einfaches Verschwenken in die jeweils gewünschte Position gebracht werden können.

Eine zweckmäßige Ausgestaltung der Erfindung besteht darin, daß die beiden Aufnahmetische unterhalb der Kompressionsvorrichtung um eine parallel zum Zentralstrahl der Röntgenstrahlenquelle liegende Achse schwenkbar gelagert sind, derart, daß sie sich um 180° gegenüberliegen. Bei dieser Ausgestaltung kann der jeweils gewünschte Aufnahmetisch durch Schwenken auf der mit dem Röntgenstrahler verbundenen Achse in die jeweilige Aufnahme- bzw. Parkposition gebracht werden. Eine andere Ausgestaltung der Erfindung besteht darin, daß die beiden Aufnahmetische mit einem um eine senkrecht zum Zentralstrahl der Röntgenstrahlenquelle liegende Achse schwenkbaren Träger eine U-förmige Aufnahmeeinheit bilden, die die Röntgenstrahlenquelle und die Kompressionsvorrichtung außen umgreift und in der sich die Aufnahmetische gegenüberliegen. Bei dieser Ausgestaltung genügt ein Schwenken der Aufnahmetische um die senkrecht zum Zentralstrahl der Röntgenstrahlenquelle liegende Achse, die auch die Röntgenstrahlenquelle trägt, um die Aufnahmetische in ihre jeweilige Position zu bringen. Um dem Wunsch der Ärzte Rechnung zu tragen, wahlweise Röntgenaufnahmen mit oder ohne Streustrahlenraster anzufertigen, können die Aufnahmetische derart ausgebildet sein, daß das dort vorgesehene Streustrahlenraster in eine Stellung bringbar ist, in der es außerhalb der Röntgenstrahlung liegt.

Die Erfindung ist nachfolgend anhand der Zeichnung näher erläutert. Es zeigen :

Figuren 1 und 2 zwei Ausführungsbeispiele eines Röntgendiagnostikgerätes nach der Erfindung, und

Figur 3 eine Einzelheit der Röntgendiagnostikgeräte gemäß den Figuren 1 und 2.

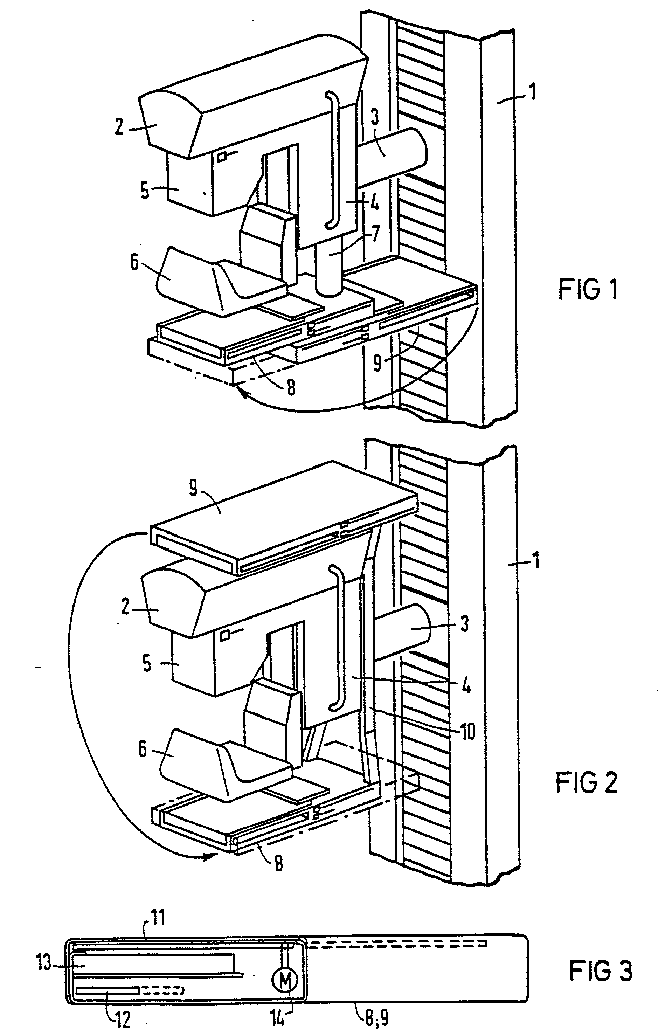

In der Figur 1 ist ein Stativ 1 dargestellt, mit dem eine Röntgenstrahlenquelle 2 mit Hilfe einer senkrecht zu deren Zentralstrahl liegenden Achse 3 höhenverstellbar ist. Die Achse 3 trägt die Röntgenstrahlenquelle 2 mit Hilfe eines Blockes 4, an dem eine Primärstrahlenblende 5 angeordnet ist. Der Block 4 trägt ferner eine motorisch in Richtung des Zentralstrahles der Röntgenstrahlenquelle 2 verstellbare Kompressionsvorrichtung 6. Achse 3 und Block 4 bilden zusammen eine Halterung für die Röntgenstrahlenquelle 2.

Parallel zum Zentralstrahl der Röntgenstrahlenquelle 2 ist am Block 4 eine Achse 7 befestigt, um die zwei Aufnahmetische 8 und 9 für unterschiedliche Kassettenformate schwenkbar gelagert sind. In der Figur 1 befindet sich der Aufnahmetisch 8 in der Aufnahmeposition, während der Aufnahmetisch 9 in der Parkposition liegt. Soll mit dem Aufnahmetisch 9 gearbeitet werden, so werden die Aufnahmetische 8 und 9 gemeinsam um die Achse 7 so verschwenkt, daß der Aufnahmetisch 9 die Aufnahmeposition und der Aufnahmetisch 8 die Parkposition einnimmt.

Bei dem Ausführungsbeispiel gemäß Figur 2 sind mit dem Ausführungsbeispiel gemäß Figur 1 gleiche Teile mit gleichen Bezugszeichen bezeichnet. Die beiden Aufnahmetische 8 und 9, die sich bei dem Ausführungsbeispiel gemäß Figur 1 um 180° gegenüberliegen, bilden bei dem Ausführungsbeispiel gemäß Figur 2 zusammen mit einem um die senkrecht zum Zentralstrahl der Röntgenstrahlenquelle liegende Achse 3 schwenkbaren Träger 10 eine U-förmige Aufnahmeeinheit. Die Aufnahmeeinheit 8, 9, 10 umgreift die Röntgenstrahlenquelle 2 und die Kompressionsvorrichtung 6 außen, und die Aufnahmetische 8 und 9 liegen sich gegenüber. In der Figur 2 befindet sich dabei der Aufnahmetisch 8 in der Aufnahmeposition, während der Aufnahmetisch 9 die Parkposition oberhalb der Röntgenstrahlenquelle 2 einnimmt. Soll der Aufnahmetisch 9 in die Aufnahmeposition bewegt werden, so wird der Träger 9 auf der Achse 3 um 180° geschwenkt, so daß er die Stellung des Aufnahmetisches 8 und der Aufnahmetisch 8 die Stellung des Aufnahmetisches 9 einnimmt.

Die Aufnahmetische 8 und 9 weisen jeweils ein Streustrahlenraster 11 sowie die Strahlenmeßkammer 12 eines Belichtungsautomaten auf (Fig. 3). Die jeweilige Röntgenfilmkassette 13 wird gemäß Figur 3 zwischen dem Streustrahlenraster 11 und der Strahlenmeßkammer 12 eingeschoben. Das Streustrahlenraster 11 wird während einer Aufnahme mit Hilfe eines Motors 14 periodisch hin- und herbewegt, um eine Abbildung auf dem Röntgenfilm zu verhindern. Die Figur 3 zeigt, daß die Aufnahmetische 8, 9 so ausgebildet sind, daß das Streustrahlenraster 11 in eine gestrichelt gezeichnete Stellung bringbar ist, in der es außerhalb der Röntgenstrahlung liegt, so daß auch Röntgenaufnahmen ohne Streustrahlenraster angefertigt werden können. Die Bewegung des Streustrahlenrasters 11 in die gestrichelt gezeichnete Stellung übernimmt zweckmäßigerweise ebenfalls der Motor 14, der hierzu geeignet gesteuert ist.

Zur Anfertigung einer Röntgenaufnahme wird bei den Ausführungsbeispielen gemäß den Figuren 1 und 2 die Brust auf dem Aufnahmetisch, der sich in der Aufnahmeposition befindet — bei den Beispielen ist dies der Aufnahmetisch 8 —, gelagert und anschließend durch Verstellung der Kompressionsvorrichtung 6 nach unten komprimiert. Anschließend erfolgt die Einschaltung des Röntgenstrahlers 2, danach die Rückbewegung der Kompressionsplatte 6 nach oben und schließlich die Entfernung der belichteten Röntgenfilmkassette 13 aus dem jeweiligen Aufnahmetisch.

**Patentansprüche**

1. Röntgendiagnostikgerät für Mammographieaufnahmen mit einer verstellbaren, an einer Halterung (3, 4) befestigten Röntgenstrahlenquelle (2), einer in Richtung des Zentralstrahles der Röntgenstrahlenquelle (2) verstellbaren Kompressionsvorrichtung (6) und mehreren, wahlweise auf den Röntgenstrahler (2) ausrichtbaren Aufnahmetischen (8, 9), dadurch gekennzeichnet, daß zwei unterschiedliche Aufnahmetische (8, 9) für unterschiedliche Kassettenformate derart schwenkbar mit der Halterung (3, 4) verbunden sind, daß jeweils einer in eine Aufnahmeposition in der Röntgenstrahlung und der zweite in eine Parkposition außerhalb der Röntgenstrahlung verstellbar ist.

2. Röntgendiagnostikgerät nach Anspruch 1, dadurch gekennzeichnet, daß die beiden Aufnahmetische (8, 9) unterhalb der Kompressionsvorrichtung (6) um eine parallel zum Zentralstrahl der Röntgenstrahlenquelle (2) liegende Achse (7) schwenkbar gelagert sind, derart, daß sie sich um 180° gegenüberliegen.

3. Röntgendiagnostikgerät nach Anspruch 1, dadurch gekennzeichnet, daß die beiden Aufnahmetische (8, 9) zusammen mit einem um eine senkrecht zum Zentralstrahl der Röntgenstrahlenquelle (2) liegende Achse (3) schwenkbaren Träger (10) eine U-förmige Aufnahmeeinheit (8, 9, 10) bilden, die die Röntgenstrahlenquelle (2) und die Kompressionsvorrichtung (6) außen umgreift, und in der sich die Aufnahmetische (8, 9) gegenüberliegen.

4. Röntgendiagnostikgerät nach einem der Ansprüche 1 bis 3, mit einem Streustrahlenraster (11) in jedem Aufnahmetisch (8, 9) dadurch gekennzeichnet, daß jeder Aufnahmetisch (8, 9) derart ausgebildet ist, daß sein Streustrahlenraster (11) in eine Stellung bringbar ist, in der es außerhalb der Röntgenstrahlung liegt.

**Claims**

1. An X-ray diagnostic apparatus adapted to mammography purposes, comprising an adjustable X-radiation source (2) attached to a mounting (3, 4), a compression device (6) which is adjustable in the direction of the central ray of the X-radiation source (2), and a plurality of supporting tables (8, 9) which can be selectively aligned with the X-radiation source (2), characterised in that two different supporting tables (8, 9) for different cassette formats are pivoted to the mounting (3, 4) in such a manner that while one can be brought into a photographing position in the X-radiation, the second is brought into a park position outside of the X-radiation.

2. An X-ray diagnostic apparatus as claimed in Claim 1, characterised in that the two supporting tables (8, 9) are mounted beneath the compression device (6) so as to be pivotable about an axis (7), extending parallel to the central ray of the X-radiation source (2), in such manner that they face one another at an angle of 180°.

3. An X-ray diagnostic apparatus as claimed in Claim 1, characterised in that the two supporting tables (8, 9), together with a carrier (10), which is pivotable about an axis (3) extending at right angles to the central ray of the X-radiation source (2), form a U-shaped supporting unit (8, 9, 10) which externally embraces the X-radiation source (2) and the compression device (6) and in which the supporting tables (8, 9) are arranged opposite one another.

4. An X-ray diagnostic apparatus as claimed in one of Claims 1 to 3, comprising a radiation scattering screen (11) in each supporting table (8, 9), characterised in that each supporting table (8, 9) is designed to be such that its radiation scattering screen (11) can be brought into a position in which it lies outside of the X-radiation.

**Revendications**

1. Appareil de radiodiagnostic pour des prises de vue mammographiques, comportant une source de rayons X (2) déplaçable et fixée sur un support (3, 4), un dispositif de compression (6) réglable dans la direction du rayon central de la source de rayons X (2) et plusieurs tables de prise de vue (8, 9) susceptibles d'être, au choix, alignées sur un générateur de rayons X (2), caractérisé par le fait que deux tables différentes de prises de vue (8, 9) pour des formats de cassettes différentes sont reliées à articulation avec le support (3, 4) de façon que l'une d'elles soit déplaçable dans une position de prise de vue dans le rayonnement X, alors que la seconde est susceptible d'être déplacée dans une position d'attente, en dehors du rayonnement X.

2. Appareil de radiodiagnostic selon la revendication 1, caractérisé par le fait que les deux tables de prises de vue (8, 9) sont montées à articulation au-dessous du dispositif de compression (6), autour d'un axe (7) qui est parallèle au rayon central de la source de rayons X (2), et cela de telle façon qu'elles se situent l'une en face de l'autre d'un angle de 180°.

3. Appareil de radiodiagnostic selon la revendication 1, caractérisé par le fait que les deux tables de prises de vue (8, 9) forment, avec un support (10) qui est articulé autour d'un axe (3) qui se situe perpendiculairement au rayon central de la source de rayons X (2), une unité de prise de vue en forme de U (8, 9, 10) qui entoure la source de rayons X (2) et le dispositif de compression (6) de l'extérieur et dans laquelle les tables de prises de vue (8, 9) se situent l'une en face de l'autre.

4. Appareil de radiodiagnostic selon l'une des revendications 1 à 3, avec une grille antidiffusante (11) dans chaque table de prises de vue (8, 9), caractérisé par le fait que chaque table de prises de vue (8, 9) est réalisée de telle façon que sa grille antidiffusante (11) est susceptible d'être amenée dans une position dans laquelle elle se situe en dehors du rayonnement X.

0 127 073

FIG 1

FIG 2

FIG 3